# EUROPEAN PATENT SPECIFICATION

(11) **EP 3 231 428 B1**
(45) Date of publication and mention of the grant of the patent: **04.03.2020**
(21) Application number: 15866838.4
(22) Date of filing: 11.12.2015
(51) Int. Cl.: A61K 31/551, A61K 31/498, A61K 45/00, A61K 47/22, A61P 27/06, A61P 43/00

(54) **PHARMACOTHERAPY FOR PREVENTING OR TREATING GLAUCOMA**
ARZNEIMITTELTHERAPIE ZUR PRÄVENTION ODER BEHANDLUNG VON GLAUKOMEN
PHARMACOTHÉRAPIE POUR LA PRÉVENTION OU LE TRAITEMENT DU GLAUCOME

(30) Priority: 12.12.2014 JP 2014252052
(43) Date of publication of application: 18.10.2017
(73) Proprietor: Kowa Company, Ltd., Nagoya-shi, Aichi-ken 460-8625 (JP)
(72) Inventor: KANEKO, Yoshio, Higashimurayama-shi Tokyo 189-0022 (JP); OHTA, Masayuki, Higashimurayama-shi Tokyo 189-0022 (JP)
(74) Representative: Schiener, Jens
(86) International application number: PCT/JP2015/084817
(87) International publication number: WO 2016/093348

(56) References cited:
- EP-A1- 1 818 059
- EP-A1- 3 231 429
- WO-A1-2006/068208
- JP-A- 2012 250 953
- JP-A- 2014 520 895
- US-A1- 2011 178 145
- TADASHIRO SAEKI ET AL: "Mouse Gan'atsu ni Taisuru ROCK Sogaizai K115 to Brimonidine no Heiyo Koka [Effect of combination of ROCK inhibitor K 15 and brimonidine on mouse intraocular pressure]", JAPANESE SOCIETY FOR OCULAR PHARMACOLOGY PROGRAM.KEEN SHOROKUSHU, JAPANESE SOCIETY FOR OCULAR PHARMACOLOGY, JP, vol. 27, 1 January 2007 (2007-01-01), page 59, XP009503706,
- LEE ANNE J ET AL: "Emerging drugs for ocular hypertension", EXPERT OPINION ON EMERGING DRUGS ENG, INFORMA HEALTHCARE, UK, vol. 16, no. 1, 1 March 2011 (2011-03-01), pages 137-161, XP008161576, ISSN: 1744-7623, DOI: 10.1517/14728214.2011.521631
- TADASHIRO SAEKI ET AL.: 'Mouse Gan'atsu ni Taisuru ROCK Sogaizai K115 to Brimonidine no Heiyo Koka' JAPANESE SOCIETY FOR OCULAR PHARMACOLOGY PROGRAM·KEEN SHOROKUSHU vol. 27, 2007, page 59, XP009503706
- GOMI NORIAKI ET AL.: 'A Practical Synthesis of (S)-tert-Butyl-3-Methyl-1,4-diazepane-1- carboxylate, the Key Intermediate of Rho-Kinase Inhibitor K-115' SYNTHESIS vol. 44, no. 20, 01 January 2012, pages 3171 - 3178, XP055453983 DOI: 10.1055/S-0032-1316771

## Description

### [Technical Field]

The present invention relates to pharmacotherapy for preventing or treating glaucoma or ocular hypertension. The invention relates to a combination of (S)-(-)-1-(4-fluoro-5-isoquinolinesulfonyl)-2-methyl-1,4-homopiperazine or a salt or solvate thereof, and brimonidine, for use in preventing or treating glaucoma or ocular hypertension, as defined in the claims.

### [Background Art]

Glaucoma is a disease which increases intraocular pressure due to various pathogenic factors and damages the optic nerve to atrophy, and results in visual field abnormality and low vision. Since the optic nerve once atrophied does not recover, leaving glaucoma untreated leads to blindness. In addition, even when glaucoma is successfully treated, the condition is only maintained as it is and expected not to be recovered. Thus, glaucoma is a refractory disease. Ocular hypertension, which is not associated with visual field abnormality but likely to develop into glaucoma over a long period, has the same risk.

Glaucoma is classified into three types: congenital glaucoma, secondary glaucoma, and primary glaucoma. Congenital glaucoma is caused by inhibition of discharge of the aqueous humor in association with natural goniodysgenesis. Secondary glaucoma is attributable to an apparent cause such as inflammation and injury, and develops due to an ocular cause such as uveitis and eye injury, and even develops due to hemorrhage associated with diabetes mellitus and long-term use of steroid hormone for treatment of another disease. Primary glaucoma, which is a collective term for glaucoma of an unknown cause, is the most common type of glaucoma and found most often in middle-aged and elderly individuals. Primary glaucoma and secondary glaucoma are further classified into two types: open-angle glaucoma and closed-angle glaucoma, on the basis of the mode of blocking of the aqueous humor flow. Although there are many patients presenting with normal-tension glaucoma, which is not associated with increase of intraocular pressure, the first goal of treatment of glaucoma is reduction of intraocular pressure.

When intraocular pressure cannot be controlled by using a drug, or a patient with closed-angle glaucoma has an acute attack of glaucoma, laser treatment (laser trabeculoplasty), surgery therapy (trabeculectomy and trabeculotomy), or the like is conducted as a method for treating glaucoma; however, pharmacotherapy is used as the first-line therapy. In pharmacotherapy for glaucoma, for example, sympathomimetics (non-selective agonists such as epinephrine, and α₂ agonists such as apraclonidine and brimonidine), sympatholytics (β-blockers such as timolol, befunolol, carteolol, nipradilol, betaxol, levobunolol, and metipranolol, α₁-blockers such as bunazosin hydrochloride), parasympathomimetics (e.g., pilocarpine), carbonic anhydrase inhibitors (e.g., acetazolamide), and prostaglandins (e.g., isopropyl unoprostone, latanoprost, travoprost, bimatoprost, and tafluprost) are used.

Among these agents, brimonidine is an agent which reduces intraocular pressure through reduction of the aqueous humor production and promotion of aqueous humor outflow via the uveoscleral pathway, and is commonly used in clinical practice (Non Patent Literature 1).

On the other hand, Rho kinase inhibitors have been found as a candidate for a therapeutic agent for glaucoma based on a novel mechanism of action (Patent Literatures 1 and 2). The Rho kinase inhibitor reduces intraocular pressure through promotion of aqueous humor outflow from the trabecula pathway (Non Patent Literature 2), and it has been suggested that the action is caused by the change of the cytoskeleton of the trabecular cell (Non Patent Literature 2 and Non Patent Literature 3).

In addition, agents having intraocular pressure-reducing action are used in combination for glaucoma or ocular hypertension for the purpose of enhancing the intraocular pressure-lowering action. For example, Non Patent Literature 4 describes combined use of intraocular pressure-lowering agents based on various mechanisms of action. In a report that the intraocular pressure-lowering action of combined use of the Rho inhibitor K-115 and the α₂ agonist brimonidine was evaluated by using mice (Non Patent Literature 5), combined use under the condition that K-115 was administered 1.5 hours after administration of brimonidine did not provide significant intraocular pressure-lowering action in comparison to single administration of K-115.

The therapeutic agents and therapeutic methods for glaucoma or ocular hypertension are still unsatisfactory in terms of the intensity and duration of intraocular pressure-lowering effect. In particular, extremely high intraocular pressure may be generated under a special condition such as an attack of glaucoma in a patient with closed-angle glaucoma, neovascular glaucoma, and postoperative condition, and in such a situation it is required to immediately decrease the intraocular pressure to prevent the optic nerve cells from being disordered. In such a circumstance, a therapeutic agent for glaucoma having more potent intraocular pressure-lowering effect with enhanced fast-acting properties is demanded.

### [Citation List]

### [Patent Literature]

[Patent Literature 1] WO00/09162
[Patent Literature 2] JP-A-11-349482

[Non Patent Literature 1] Arch. Ophthalmol., 113 (12), 1514-1517 (1995)
[Non Patent Literature 2] IOVS, 42 (1), 137-144 (2001)
[Non Patent Literature 3] IOVS, 42 (5), 1029-1037 (2001)
[Non Patent Literature 4] Exp. Opin. Emer. Drugs, 12 (2), 313-327 (2007)
[Non Patent Literature 5] Proceedings of the 27th Annual Scientific Meeting of the Japanese Society for Ocular Pharmacology, Oral presentation No. 18, 59 (2007)

### [Summary of the Invention]

The invention is defined by the claims. Any subject-matter falling outside the scope of the claims is provided for information purposes only. Any references in the description to methods of treatment refer to the compounds, pharmaceutical compositions and medicaments of the present invention for use in a method for treatment of the human or animal body by therapy.

### [Technical Problem]

The present invention relates to provision of a pharmacotherapy for preventing or treating glaucoma or ocular hypertension, the pharmacotherapy providing potent intraocular pressure-lowering action with fast-acting properties and prolonged duration.

### [Solution to Problem]

The present inventors conducted extensive research to solve the above-described problem, and found that administration of (S)-(-)-1-(4-fluoro-5-isoquinolinesulfonyl)-2-methyl-1,4-homopiperazine (hereinafter, occasionally referred to as Compound 1) or a salt thereof, or a solvate of Compound 1 or the salt thereof in combination with brimonidine as an α₂ agonist provides potent intraocular pressure-lowering action with fast-acting properties and prolonged duration. It is reported that, in the case of rabbits, the α₂ agonist causes transient intraocular pressure-increasing action based on stimulation of the α₁ adrenergic receptor present in the peripheral nerve synapse prior to intraocular pressure-lowering action in an eye to which the α₂ agonist was applied, and then reduction of the aqueous humor production and increase of the aqueous humor outflow from the uveoscleral pathway via the adrenergic α₂ receptor present in the eye result in decreased intraocular pressure (Current eye research, 5 (9), 665-676 (1986), Ann N Y Acad Sci, 763, 78-95 (1995)). Accordingly, it was a quite unexpected result that a combination of brimonidine and Compound 1 provided potent intraocular pressure-lowering action in a short time.

In summary, the present invention relates to the following invention.
1) A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating glaucoma, wherein the α₂ agonist is brimonidine or a salt thereof, and being a combination drug incorporated in a single dosage form.
2) A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating ocular hypertension, wherein the α₂ agonist is brimonidine or a salt thereof, and being a combination drug incorporated in a single dosage form.
3) A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating glaucoma or for use in preventing or treating ocular hypertension, wherein the α₂ agonist is brimonidine or a salt thereof, being a kit configured such that the agents are independently formulated; and wherein the use comprises simultaneous administration of the agents.
4) A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating glaucoma or for use in preventing or treating ocular hypertension, wherein the α₂ agonist is brimonidine or a salt thereof, being a kit, and wherein the use comprises administering a formulation comprising (S)-(-)-1-(4-fluoro-5-isoquinolinesulfonyl)-2-methyl-1,4-homopiperazine as a first agent and then administering a formulation comprising brimonidine or a salt thereof as a second agent.

### [Effects of the Invention]

In accordance with the present invention, a means for preventing or treating glaucoma or ocular hypertension as defined in the claims can be provided, the means providing potent intraocular pressure-lowering action with fast-acting properties and prolonged duration.

### [Brief Description of Drawing]

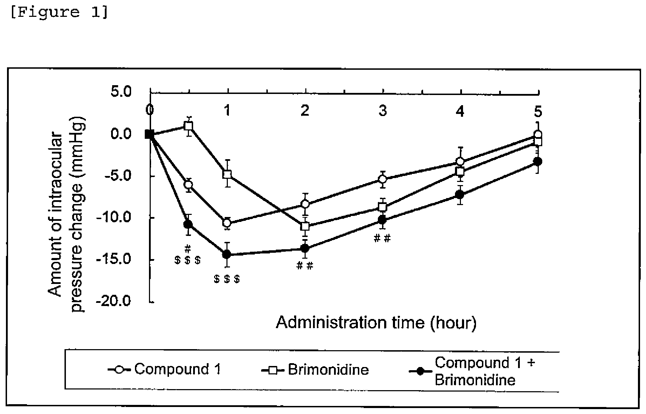

[Figure 1] Figure 1 is a graph showing the temporal variation of intraocular pressure for different administration groups. Intraocular pressure is represented as an amount of change from initial intraocular pressure (average value ± standard deviation). □: a group receiving single administration of brimonidine; ○: a group receiving single administration of (S)-(-)-Compound 1; **●:** a group receiving combined administration of brimonidine and Compound 1; statistical analysis: # p<0.05, ## p<0.01 vs. Compound 1 group, $$$ p<0.001 vs. brimonidine group.

### [Description of Embodiments]

(S)-(-)-1-(4-fluoro-5-isoquinolinesulfonyl)-2-methyl-1,4-homopiperazine used in the present invention (Compound 1) is a compound having antagonistic action on substance P, antagonistic action on leukotriene D4, and Rho kinase inhibitory action, and can be produced using a known method, for example, a method described in WO99/20620.

Examples of salts of Compound 1 include salts of inorganic acids such as hydrochloric acid, sulfuric acid, nitric acide, hydrofluoric acid, and hydrobromic acid; and salts of organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid, with hydrochloride being particularly preferred.

Compound 1 or a salt thereof may be present not only as an unsolvated form but also as a hydrate or a solvate. Although a hydrate is preferred, all the crystalline forms and hydrates and solvates are contemplated in the present invention.

The α₂ agonist is only required to have intraocular pressure-lowering action and be useful for treatment of glaucoma. Examples of α₂ agonists having intraocular pressure-lowering action include clonidine, apraclonidine, and brimonidine. For example, brimonidine can be produced using a known method, for example, described in Bioorganic & Medicinal Chemistry Letters (1995), 5(19), 2255-8.

Examples of salts of brimonidine include salts of inorganic acids such as hydrochloric acid, sulfuric acid, nitric acid, hydrofluoric acid, and hydrobromic acid; and salts of organic acids such as acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, and camphorsulfonic acid, with tartrate being particularly preferred.

Brimonidine or a salt thereof may be present not only as an unsolvated form but also as a hydrate or a solvate. Although a hydrate is preferred, all the crystalline forms and hydrates and solvates are contemplated in the present invention.

When (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof (Compound 1) is used in combination with an α₂ agonist, potent, synergetic intraocular pressure-lowering action is observed immediately after administration, as demonstrated in Example below. In addition, this potent intraocular pressure-lowering action is sustained for several hours. Thus, such combination is useful as a prophylactic or therapeutic agent for glaucoma or ocular hypertension, and such combined administration is useful as a pharmacotherapy for preventing or treating glaucoma or ocular hypertension. Examples of types of glaucoma include primary open-angle glaucoma, normal-tension glaucoma, hypersecretion glaucoma, ocular hypertension, acute closed-angle glaucoma, chronic closed-angle glaucoma, plateau iris syndrome, combined mechanism glaucoma, steroid induced glaucoma, capsular glaucoma, pigmentary glaucoma, amyloidotic glaucoma, neovascular glaucoma, and malignant glaucoma. Ocular hypertension, which is also referred to as ocular high blood pressure, is a symptom presenting as abnormally high intraocular pressure despite findings of no clear lesions in the optic nerve, and encompasses various types of high intraocular pressure condition including development of high intraocular pressure after surgery.

The combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine of the salt thereof (Compound 1) and an α₂ agonist for preventing or treating glaucoma or ocular hypertension of the present invention may be used to produce a combination drug (a prophylactic or therapeutic agent for glaucoma, or a prophylactic or therapeutic agent for ocular hypertension) by incorporating the components each in an effective amount at an appropriate incorporation ratio in a single dosage form, or may be a kit configured so that agents independently formulated and each comprising one of the components in an effective amount are used simultaneously or separately at an interval, as defined in the claims.

In the case where (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof (Compound 1) and an α₂ agonist are independently produced as a formulation, each formulation can be prepared in accordance with a known method. For example, a formulation of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof can be prepared with reference to a formulation example described in Patent Literature 1 or WO97/23222, and in the case where the α₂ agonist is brimonidine, a formulation thereof can be prepared with reference to a formulation example described in WO92/13855. For brimonidine, a commercially available formulation can be used.

In the case where a formulation incorporating (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist is prepared, the formulation can be similarly prepared in accordance with a known method. For example, an eye drop can be prepared using an isotonic agent, a buffer, a surfactant, an antiseptic, or the like, as necessary. The pH is only required to be in a range acceptable for ophthalmic formulations, and preferably in the range of pH 4 to 8.

The above formulation is preferably used as an ophthalmic formulation, in particular, an eye drop, and the eye drop may be any of an aqueous eye drop, a non-aqueous eye drop, a suspension eye drop, an emulsion eye drop, and an eye ointment. Such a formulation can be produced as a composition suitable for form of administration by incorporating a pharmaceutically acceptable carrier, for example, an isotonic agent, a chelating agent, a stabilizer, a pH regulator, an antiseptic, an antioxidant, a solubilizer, or a thickener, as necessary, in accordance with a (formulation) method known in the art.

In the case where an eye drop is prepared, for example, the above components desired are dissolved or suspended in an aqueous solvent such as sterile purified water and physiological saline, or a non-aqueous solvent such as plant oil such as cottonseed oil, soybean oil, sesame oil, and peanut oil, the osmotic pressure is adjusted to a predetermined osmotic pressure, and the resultant is subjected to sterilization such as filter sterilization. In the case where an eye ointment is prepared, an ointment base can be contained in addition to the above components. The ointment base is not particularly limited, and preferred examples thereof include oily bases such as petrolatum, liquid paraffin, and polyethylene; emulsion bases, with the oil phase and aqueous phase emulsified with a surfactant or the like; and water-soluble bases consisting of hydroxypropylmethyl cellulose, carboxymethyl cellulose, polyethylene glycol, or the like.

In the case where a combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist is used as a kit for preventing or treating glaucoma or ocular hypertension, the kit can be designed so that an agent comprising (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an agent comprising α₂ agonist, each formulated as described above, are independently packaged, and each pharmaceutical formulation is taken out of the corresponding package and used in administration. Alternatively, each pharmaceutical formulation may be packaged in a form suitable for combined administration in portions.

In the case where (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof is used in combination with an α₂ agonist for preventing or treating glaucoma or ocular hypertension, the dose varies depending on the body weight, age, sex and the symptoms of a patient, form of administration, the frequency of administration, etc., and an exemplary dose in terms of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof for an adult is in common cases 0.025 to 10,000 µg, preferably 0.025 to 2,000 µg, and more preferably 0.1 to 2,000 µg per day, and an exemplary dose in terms of an α₂ agonist for an adult is in common cases 3 to 10,000 µg, and preferably 30 to 3,000 µg per day. As a specific example for an α₂ agonist, 30 to 300 µg of brimonidine is used per day in common cases.

In the case where a formulation incorporating (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist is administered, an appropriate incorporation ratio is suitably selected to prepare a formulation so that the dose of each of the above components per day is equal to or less than the above-described dose.

While the frequency of administration is not particularly limited, it is preferred to administer singly or in several portions, and in the case of a liquid eye drop, it is suitable to apply one to several drops per administration. In the case of a kit, the individual formulations may be simultaneously administered or separately administered as defined in the claims at an interval of 5 minutes to 24 hours. In the case of separate administration at an interval, it is preferred to employ a procedure in which a formulation comprising (S)-(-)-1-(4-fluoro-5-isoquinolinesulfonyl)-2-methyl-1,4-homopiperazine is administered as a first agent, and then an agent comprising an α₂ agonist is administered as a second agent.

Hereinafter, the present invention will be described in more detail.

### [Example]

### Example 1

To investigate the usefulness of the combination of (S)-(-)-1-(4-fluoro-5-isoquinolinesulfonyl)-2-methyl-1,4-homopiperazine (Compound 1) and an α₂ agonist, intraocular pressure-lowering effect was compared between the case where each drug was singly administered to an experimental animal and the case where the drugs were administered to an experimental animal in combination.

### 1. Preparation of solution of test compound

### A. Preparation of solution of Compound 1

(S)-(-)-1-(4-fluoro-5-isoquinolinesulfonyl)-2-methyl-1,4-homopiperazine hydrochloride dihydrate was dissolved in physiological saline, and the solution was then neutralized (pH 6.0) with addition of sodium dihydrogen phosphate and sodium hydroxide to prepare a solution of Compound 1 at a desired concentration.

### B. Preparation of α₂ receptor agonist

Commercially available brimonidine (Senju Pharmaceutical Co., Ltd., AIPHAGAN eye drop 0.1%) was directly used.

### 2. Test method

The intraocular pressure-lowering effect of combined administration of Compound 1 and brimonidine was investigated. As controls, the intraocular pressure-lowering effect of single administration of Compound 1 and that of single administration of brimonidine were further investigated.

### A. Agents and animals used in test

Solution of Compound 1: 0.4% solution (amount of application: 50 µL)
Brimonidine: 0.1% eye drop (amount of application: 50 µL)
Experimental animal: white rabbit (sex: male, 10 rabbits per group)

### B. Administration method and measurement method

### (1) Combined administration of agents

1) One drop of 0.4% oxybuprocaine hydrochloride eye drop (trade name: Benoxil 0.4% solution) was applied to both eyes of an experimental animal for local anesthesia (data were acquired only from the eye to which the eye drop was applied).
2) The intraocular pressure was measured immediately before administration of a solution of a test compound, and the measurement was used as the initial intraocular pressure.
3) The solution of Compound 1 was applied to one eye of the experimental animal, and subsequently the solution of brimonidine was applied to the same eye.
4) One drop of the 0.4% oxybuprocaine hydrochloride eye drop was applied to each eye for local anesthesia 0.5 hours, 1 hour, 2 hours, 3 hours, 4 hours, and 5 hours after the application of the agents, and the intraocular pressure was then measured.

### (2) Single administration of Compound 1

Compound 1 was singly applied, and a test was performed at the same measurement times as in the combined administration test.

### (3) Single administration of brimonidine

A test was performed using the same method as in the single administration test except that the test solution in the single administration of Compound 1 was replaced with that of brimonidine.

### 3. Results and discussion

The test results are shown in Table 1 and Figure 1. The intraocular pressure in Figure 1 was represented as an amount of change from the initial intraocular pressure. For statistical processing, Stat Preclinica Client 1.1 with parametric Tukey's multiple comparison was used.

**[Table 1]**

| IOP | 0h | 0.5h | 1h |
|---|---|---|---|
| Compound 1 | 26.5±0.8 | 20.5±1.2 | 15.9±1.0 |
| Brimonidine | 24.2±1.3 | 25.2±0.9 | 19.4±1.2 |
| Combined use | 24.9±1.2 | 14.2±1.6 | 10.6±0.5 |

| | | | |
|---|---|---|---|
| n=10, IOP : intraocular pressure (mmHg) | | | |

As can be seen from Table 1, the combined use of Compound 1 and brimonidine was confirmed to provide intraocular pressure-lowering action with fast-acting properties. In particular, 0.5 hours after application of an eye drop, the combined use of the two agents brought synergetic, potent intraocular pressure-lowering action, while the single administration of brimonidine provided no intraocular pressure-lowering action. As is clear from Figure 1, the group with combined use of Compound 1 and brimonidine exhibited intraocular pressure-lowering action superior to that of the groups with single administration of an agent, i.e., the group with administration of Compound 1 or the group with administration of brimonidine, and in addition the long-lasting properties were improved.

From these results, combining Compound 1 and brimonidine was found to allow more potent intraocular pressure-lowering effect to develop at an early stage, and provide improvement of the long-lasting properties.

### [Industrial Applicability]

The combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist as defined in the claims has excellent intraocular pressure-lowering effect with fast-acting properties and long-lasting properties, and thus is useful for a medicine for preventing or treating glaucoma or ocular hypertension.

## Claims

1. A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating glaucoma, wherein the α₂ agonist is brimonidine or a salt thereof, and being a combination drug incorporated in a single dosage form.

2. A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating ocular hypertension, wherein the α₂ agonist is brimonidine or a salt thereof, and being a combination drug incorporated in a single dosage form.

3. A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating glaucoma or for use in preventing or treating ocular hypertension, wherein the α₂ agonist is brimonidine or a salt thereof, being a kit configured such that the agents are independently formulated; and wherein the use comprises simultaneous administration of the agents.

4. A combination of (S)-(-)-1-(4-fluoro-5-isoquinolinesulfonyl)-2-methyl-1,4-homopiperazine or a salt thereof, or a solvate of (S)-(-)-1-(4-fluoro-5-isoquinolinesulfonyl)-2-methyl-1,4-homopiperazine or the salt thereof and an α₂ agonist for use in preventing or treating glaucoma or for use in preventing or treating ocular hypertension, wherein the α₂ agonist is brimonidine or a salt thereof, being a kit and wherein the use comprises administering a formulation comprising (S)-(-)-1-(4-fluoro-5-isoquinolinesulfonyl)-2-methyl-1,4-homopiperazine as a first agent and then administering a formulation comprising brimonidine or a salt thereof as a second agent.

## Patentansprüche

1. Kombination von (S)-(-)-1-(4-Fluor-5-isochinolinsulfonyl)-2-methyl-1,4-homopiperazin oder eines Salzes desselben oder eines Solvats von (S)-(-)-1-(4-Fluor-5-isochinolinsulfonyl)-2-methyl-1,4-homopiperazin oder eines Salzes desselben und eines α₂-Agonisten zur Verwendung bei der Vorbeugung oder der Behandlung eines Glaukoms, wobei der α₂-Agonist Brimonidin oder ein Salz desselben ist und welches ein Kombinationspräparat ist, das in einer einzelnen Darreichungsform aufgenommen ist.

2. Kombination von (S)-(-)-1-(4-Fluor-5-isochinolinsulfonyl)-2-methyl-1,4-homopiperazin oder eines Salzes desselben oder eines Solvats von (S)-(-)-1-(4-Fluor-5-isochinolinsulfonyl)-2-methyl-1,4-homopiperazin oder eines Salzes desselben und eines α₂-Agonisten zur Verwendung bei der Vorbeugung oder der Behandlung einer okulären Hypertension, wobei der α₂-Agonist Brimonidin oder ein Salz desselben ist und welches ein Kombinationspräparat ist, das in einer einzelnen Darreichungsform aufgenommen ist.

3. Kombination von (S)-(-)-1-(4-Fluor-5-isochinolinsulfonyl)-2-methyl-1,4-homopiperazin oder eines Salzes desselben oder eines Solvats von (S)-(-)-1-(4-Fluor-5-isochinolinsulfonyl)-2-methyl-1,4-homopiperazin oder eines Salzes desselben und eines α₂-Agonisten zur Verwendung bei der Vorbeugung oder der Behandlung eines Glaukoms oder zur Verwendung bei der Vorbeugung oder der Behandlung einer okulären Hypertension, wobei der α₂-Agonist Brimonidin oder ein Salz desselben ist, welche ein Kit ist, der derart gestaltet ist, dass die Mittel unabhängig formuliert werden und wobei die Verwendung die gleichzeitige Verabreichung der Mittel umfasst.

4. Kombination von (S)-(-)-1-(4-Fluor-5-isochinolinsulfonyl)-2-methyl-1,4-homopiperazin oder eines Salzes desselben oder eines Solvats von (S)-(-)-1-(4-Fluor-5-isochinolinsulfonyl)-2-methyl-1,4-homopiperazin oder eines Salzes desselben und eines α₂-Agonisten zur Verwendung bei der Vorbeugung oder der Behandlung eines Glaukoms oder zur Verwendung bei der Vorbeugung oder der Behandlung einer okulären Hypertension, wobei der α₂-Agonist Brimonidin oder ein Salz desselben ist, welche ein Kit ist, und wobei die Verwendung die Verabreichung einer Formulierung als erstes Mittel umfasst, die (S)-(-)-1-(4-Fluor-5-isochinolinsulfonyl)-2-methyl-1,4-homopiperazin umfasst, und dann die Verabreichung einer Formulierung als zweites Mittel, die Brimonidin oder ein Salz desselben umfasst.

## Revendications

1. Combinaison de (S)-(-)-1-(4-fluoro-5-isoquinolinesulfonyl)-2-méthyl-1,4-homopipérazine ou d'un sel de celle-ci, ou d'un solvate de (S)-(-)-1-(4-fluoro-5-isoquinolinesulfonyl)-2-méthyl-1,4-homopipérazine ou de son sel, et d'un agoniste α₂, pour une utilisation dans la prévention ou le traitement d'un glaucome, dans laquelle l'agoniste α₂ est la brimonidine ou un sel de celle-ci, et qui est un médicament combiné incorporé sous une seule forme posologique.

2. Combinaison de (S)-(-)-1-(4-fluoro-5-isoquinolinesulfonyl)-2-méthyl-1,4-homopipérazine ou d'un sel de celle-ci, ou d'un solvate de (S)-(-)-1-(4-fluoro-5-isoquinolinesulfonyl)-2-méthyl-1,4-homopipérazine ou de son sel, et d'un agoniste α₂, pour une utilisation dans la prévention ou le traitement d'une hypertension oculaire, dans laquelle l'agoniste α₂ est la brimonidine ou un sel de celle-ci, et qui est un médicament combiné incorporé sous une seule forme posologique.

3. Combinaison de (S)-(-)-1-(4-fluoro-5-isoquinolinesulfonyl)-2-méthyl-1,4-homopipérazine ou d'un sel de celle-ci, ou d'un solvate de (S)-(-)-1-(4-fluoro-5-isoquinolinesulfonyl)-2-méthyl-1,4-homopipérazine ou de son sel, et d'un agoniste α₂, pour une utilisation dans la prévention ou le traitement d'un glaucome ou pour une utilisation dans la prévention ou le traitement d'une hypertension oculaire, dans laquelle l'agoniste α₂ est la brimonidine ou un sel de celle-ci, et qui est un kit configuré de façon que les agents soient formulés indépendamment ; et dans laquelle l'utilisation comprend l'administration simultanée des agents.

4. Combinaison de (S)-(-)-1-(4-fluoro-5-isoquinolinesulfonyl)-2-méthyl-1,4-homopipérazine ou d'un sel de celle-ci, ou d'un solvate de (S)-(-)-1-(4-fluoro-5-isoquinolinesulfonyl)-2-méthyl-1,4-homopipérazine ou de son sel, et d'un agoniste α₂, pour une utilisation dans la prévention ou le traitement d'un glaucome ou pour une utilisation dans la prévention ou le traitement d'une hypertension oculaire, dans laquelle l'agoniste α₂ est la brimonidine ou un sel de celle-ci, qui est un kit et dans laquelle l'utilisation comprend l'administration d'une formulation comprenant de la (S)-(-)-1-(4-fluoro-5-isoquinolinesulfonyl)-2-méthyl-1,4-homopipérazine en tant que premier agent et ensuite l'administration d'une formulation comprenant de la brimonidine ou un sel de celle-ci en tant que deuxième agent.
